(19) 

Europäisches
Patentamt
European
Patent Office
Office européen
des brevets

(11) **EP 3 101 120 A1**

(12) **EUROPEAN PATENT APPLICATION**
published in accordance with Art. 153(4) EPC

(43) Date of publication:
**07.12.2016 Bulletin 2016/49**

(21) Application number: **15743963.9**

(22) Date of filing: **29.01.2015**

(51) Int Cl.:
**C12N 5/02** (2006.01)   **C12N 5/071** (2010.01)
**C07K 16/18** (2006.01)

(86) International application number:
**PCT/KR2015/000997**

(87) International publication number:
**WO 2015/115849 (06.08.2015 Gazette 2015/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **29.01.2014 KR 20140011229**

(71) Applicant: **LG Life Sciences Ltd.
Seoul 110-783 (KR)**

(72) Inventors:
 • **JUNG, Jun**
  **Daejeon 305-738 (KR)**
 • **SONG, Won Seok**
  **Daejeon 305-738 (KR)**
 • **LEE, Jun Eok**
  **Daejeon 305-738 (KR)**
 • **KIM, Yeon Chul**
  **Daejeon 305-73 (KR)**

(74) Representative: **Awapatent AB
P.O. Box 5117
200 71 Malmö (SE)**

(54) **METHOD FOR CONTROLLING GALACTOSYLATION OF RECOMBINANT PROTEIN THROUGH OPTIMIZATION OF CULTURE MEDIUM**

(57) Disclosed are a method for preparing a target recombinant protein with modulated galactosylation or a method for modulating the galactosylation of a target recombinant protein, including increasing the osmolality of a culture solution of animal cells which express a target recombinant protein during the animal cell culture; a method for preparing a target recombinant protein with modulated galactosylation or a method for modulating the galactosylation of a target recombinant protein, including supplementing asparagine to a culture solution of animal cells which express a target recombinant protein during the animal cell culture; a method for preparing a target recombinant protein with modulated galactosylation or a method for modulating the galactosylation of a target recombinant protein, including increasing the osmolality of an animal cell culture which expresses a target recombinant protein and supplementing asparagine thereto during the animal cell culture; and a target recombinant protein with modulated galactosylation, which is prepared by the method.

[FIG. 1]

EP 3 101 120 A1

**Description**

**[Technical Field]**

The present invention relates to a method for preparing a target recombinant protein with modulated galactosylation or a method for modulating the galactosylation of a target recombinant protein, including increasing the osmolality of a culture solution of animal cells which express a target recombinant protein during the animal cell culture; a method for preparing a target recombinant protein with modulated galactosylation or a method for modulating the galactosylation of a target recombinant protein, including supplementing asparagine to a culture solution of animal cells which express a target recombinant protein during the animal cell culture; a method for preparing a target recombinant protein with modulated galactosylation or a method for modulating the galactosylation of a target recombinant protein, including increasing the osmolality of an animal cell culture which expresses a target recombinant protein, and supplementing asparagine thereto during the animal cell culture; and a target recombinant protein with modulated galactosylation, which is prepared by the method.

**[Background Art]**

Antibodies are proteins which bind to antigens thereby interfering with the actions of the antigens or removing the antigens. As the therapeutic antibody market is known to have a significant potential, intensive studies are conducted on the efficient expression and large-scale production of antibodies. One of the important points in producing antibodies is the homogeneity of the population of antibodies prepared. In particular, in producing monoclonal antibodies, glycoform profiles are important for constant and reproducible monoclonal antibodies. However, many different variables can affect the glycated profiles of the produced monoclonal antibodies, and thus, there has been a need for the development of a method capable of constantly controlling the glycosylation of the prepared population of antibodies and the content of antibody isomers.

One of the important issues in commercial production of monoclonal antibodies is to constantly maintain the quality of monoclonal antibodies produced in each batch. Although there are numerous quality attributes during the process of monoclonal antibody production, the galactosylation of antibody may be one of the most important qualities among them. This is because galactosylation is known to affect the process of complement dependent cytotoxicity (CDC), which is one of the highest modes of action (MOA) of monoclonal antibodies. Galactosylation is established using galactose as a constitutional unit for the glycosylation chain reaction by linking it next to a N-acetylglucosamine sugar via galactosyltransferase. Examples of the methods for controlling galactosylation may include a method of adding manganese or galactose to a culture solution, etc. (U.S. Patent Application Publication No. 2012-0276631), but there is still a need for the development of a method for controlling galactosylation of antibodies.

**[Disclosure]**

**[Technical Problem]**

Under these circumstances, the present inventors have made many efforts to develop a method for effectively controlling the galactosylation of recombinant proteins prepared during the process of culturing animal cells which can produce the recombinant proteins. As a result, the inventors have discovered that the galactosylation of recombinant proteins can be effectively modulated by increasing the osmolality of a culture solution of animal cells or supplementing asparagine at a particular time point of the culturing process during the process of culturing the animal cells. Additionally, the inventors have discovered that when asparagine is supplemented simultaneously with an increase of the osmolality of the culture solution of animal cells, the galactosylation can be controlled without reduction in the content of the acidic antibody isomers according to the osmolality increase, thereby completing the present invention.

**[Technical Solution]**

An object of the present invention is to provide a method for preparing a target recombinant protein with modulated galactosylation, including increasing the osmolality of a culture solution of animal cells which express the target recombinant protein during the process of culturing the animal cells.

Another object of the present invention is to provide a method for modulating the galactosylation of a target recombinant protein, including increasing the osmolality of a culture solution of animal cells which express the target recombinant protein during the process of culturing the animal cells.

Still another object of the present invention is to provide a method for preparing a target recombinant protein with modulated galactosylation, including supplementing asparagine to a culture solution of animal cells which express the target recombinant protein, during the process of culturing the animal cells.

Still another object of the present invention is to provide a method for modulating the galactosylation of a target recombinant protein, including supplementing asparagine to a culture solution of animal cells which express the target recombinant protein during the process of culturing the animal cells.

Still another object of the present invention is to provide a method for preparing a target recombinant protein with modulated galactosylation, including increasing the osmolality of a culture solution of animal cells which express the target recombinant protein, and supplementing asparagine thereto during the process of culturing the animal cells.

Still another object of the present invention is to provide a method for modulating the galactosylation of a target recombinant protein, including increasing the osmolality of a culture solution of animal cells which express the target recombinant protein, and supplementing asparagine thereto during the process of culturing the animal cells.

Still another object of the present invention is to provide a target recombinant protein prepared by the above method.

**[Advantageous Effects of the Invention]**

The method of the present invention has an advantage in that the galactose content of a target recombinant protein can be effectively modulated to a desired range. Additionally, the method of the present invention, which employs both the increase of osmolality and the addition of asparagine, has the effect of modulating the content of acidic antibody isomers (acidic variants) as well as galactose. Accordingly, the method of the present invention can be effectively used for the preparation of a desired population of antibodies.

**[BRIEF DESCRIPTION OF THE DRAWINGS]**

FIG. 1 shows a schematic diagram of an expression vector of adalimumab biosimilar.
FIG. 2 shows a time point for injecting an aqueous sodium chloride (NaCl) solution during the period of a fed-batch culture.
FIG. 3 shows a time point for supplementing asparagine during the period of a fed-batch culture.

**[BEST MODE]**

In order to achieve the above objects, an aspect of the present invention provides a method for preparing a target recombinant protein with modulated galactosylation, including increasing the osmolality of a culture solution of animal cells which express the target recombinant protein during the process of culturing the animal cells.

Specifically, the above method is a method for preparing a target recombinant protein with modulated galactosylation, including increasing the osmolality of a culture solution of animal cells which express a target recombinant protein on a particular day in culture during the process of culturing the animal cells.

In the present invention, the culture may be a fed-batch culture, but is not limited thereto.

As used herein, the term "fed-batch culture" refers to a cell culture in which the initial cell culture is started by a basal medium or a production medium and the culture is continued while a feeding medium is added continuously or discontinuously to the culture at any time during the cell growth phase or antibody-production phase.

As used herein, the term "cell culture medium" or "culture medium" refers to a nutrient solution for maintenance, growth, proliferation, or expansion of cells in an artificial *in vitro* environment, which is an external side of a multicellular organism or tissue. The cell culture medium may be optimized for culturing particular cells, e.g., a basal medium prepared for supporting cell growth, or a production medium prepared for promoting the production of monoclonal antibodies, and a concentrated medium prepared by concentrating nutrients at high concentration. Nutrients and medium components refer to the components that constitute a cell culture medium, and they can be interchangeably used in the present invention.

Specifically, "basal culture medium" or "basal medium" refers to a medium which can support the minimal growth of cells. The basal medium not only provides standard inorganic salts, e.g., zinc, iron, magnesium, calcium, and potassium, but also provides trace elements, vitamins, energy sources, buffers, and amino acids. Examples of the basal medium include Dulbecco's Modified Eagle Medium (DMEM), Basal Medium Eagle (BME), RPMI 1640, F-10, and F-12, but are not limited thereto.

Additionally, specifically, the terms "cell culture production medium" or "production medium" refer to a medium which is used for the purpose of optimizing the expression of monoclonal antibodies in a bioreactor. The composition of production medium may be the same as or different from that of the basal medium, and when it is different, the production medium may be prepared by concentrating the basal medium or adding particular components to the basal medium.

As used herein, the terms "feeding medium" and "additional culture medium" may refer to a medium composed of a particular nutrient or a plurality of nutrients, which are both concentrated components of basal media. The components and concentrations of the feeding media may be prepared variously according to the cells to be cultured.

In the above culture process, the cell growth phase refers to a period during which cells grow rapidly after inoculation. In the case of Chinese hamster ovary (CHO) cells, it is known that the number of cells increases most actively at a temperature of 35°C to 37°C and at a pH of 7.0 to 7.3. In the present invention, the culture parameters during the cell growth phase were 36.5°C and at pH 7.1 or pH 7.0.

The term "inoculation" refers to a seeding of cells into a medium, which is supplied for cell culture. In particular, the medium may be supplied before transferring the cells or may be supplied simultaneously with the cells into a cell bioreactor. In a large-scale animal cell culture, conventionally, a medium is supplied in advance and cells are transferred into a bioreactor after maintaining temperature and oxygen saturation in a predetermined range.

The term "antibody-production phase" refers to a period during which the changes in process are applied for the optimization of monoclonal antibody production. In particular, the changes in process for optimizing the production may include all of lowering temperature, shift of dissolved oxygen, and media change.

In the present invention, it was confirmed that the degree of changes in the galactosylation of recombinant proteins produced in animal cells which express recombinant proteins can vary according to the time point when osmolality of media is increased. That is, when the osmolality was gradually increased during the culture period, the difference in the degree of galactosylation was not significant compared to that of the group in which the osmolality was not increased. In contrast, when the osmolality was increased at a particular time point during the culture, the G0F content was significantly increased, thus confirming that galactosylation can be modulated, and specifically, galactosylation can be prevented or reduced.

Additionally, in the present invention, the step of increasing osmolality is preferably performed during the main culture process of animal cells which express a target recombinant protein, but is not limited thereto.

As used herein, the term "main culture" refers to a step of culture in which the actual production of monoclonal antibodies is accomplished. It is the final step of the culture process which had started with a single vial of cells, and upon completion of the main culture, the monoclonal antibodies produced are subjected to purification. The main culture can be distinguished from the term called "seed culture", which is used for the purpose of stepwise increase of a culture volume.

The step of increasing osmolality may be performed at a particular time point of culture during the main culture process of animal cells; specifically, on any one day in culture during the main culture of day 1 to day 10, based on day 0 as the start of the main culture; more specifically, on any one day in culture among day 1, day 4, day 7, and day 10, based on day 0 as the start of the main culture; and more specifically, on day 4 or day 7, based on day 0 as the start of the main culture, but is not limited thereto.

Additionally, the step of increasing osmolality may be performed a single time during the process of culturing the animal cells which express the target recombinant protein.

Additionally, the step of increasing osmolality, although not particularly limited thereto, may be performed to have the final osmolality in a culture solution to be in the range of 460 mOsm/kg to 500 mOsm/kg, and more specifically, in the range of 460 mOsm/kg to 480 mOsm/kg, but is not limited thereto. The final osmolality in the culture solution refers to the osmolality appearing at the time point of culture termination, but is not particularly limited thereto.

Additionally, the step of increasing osmolality may have a target osmolality increase in a culture solution to be in the range of 400 mOsm/kg to 440 mOsm/kg, and more specifically, in the range of 430 mOsm/kg to 440 mOsm/kg, but is not limited thereto. The target osmolality increase in the culture solution refers to the level of osmolality to be increased to at the time point of performing the step of increasing osmolality, but is not particularly limited thereto.

Additionally, the increase of the osmolality may be performed using at least one method selected from the group consisting of a method of supplementing sodium chloride or potassium chloride to a culture solution of the animal cells and a method of adding glucose to the culture solution, and more specifically, using a method to supplement sodium chloride to the culture solution, but is not limited thereto.

The method of supplementing sodium chloride to a culture solution may be performed by adding a 4 M aqueous sodium chloride solution to the culture solution on a particular day during the progress of a fed-batch culture, thereby increasing the osmolality to a certain level, but is not limited thereto. When the osmolality in a culture solution, or even further, the osmolality in cells, is increased by the addition of an aqueous sodium chloride solution, it results in the increase of $\beta$-galactosidase activity, and thus the galactosylation of monoclonal antibodies may be inhibited.

As used herein, the term "culture broth" or "culture medium" refers to a liquid containing culturing cells, which is included in a shake flask or bioreactor. The culture broth and culture medium may be used interchangeably. Additionally, the culture solution and the culture medium may be distinguishable depending on the presence of animal cells.

As used herein, the recombinant protein may be an antibody, and preferably a monoclonal antibody.

As used herein, the term "monoclonal antibody" refers to an antibody which can be formed by a cell with antibody-encoding sequence and recognizes a specific antigen.

The antibody of the present invention, although not limited thereto, may preferably include all therapeutic antibodies conventionally used in the art, and specifically, it may be adalimumab.

Additionally, the above antibody is a concept encompassing both full length antibodies and antibody fragments, and examples of the antibody fragments include all of Fv, Fab, Fab', F(ab')$_2$, Fd, etc. The Fv includes both forms of disulfide-stabilized Fv (dsFv) and single chain Fv (scFv). Fd refers to the heavy chain component included in Fab.

The animal cells which can express the target recombinant protein may include a native type or transfected cell capable of expressing the recombinant protein without limitation. For the purpose of the present invention, the animal cells may be capable of expressing the recombinant protein which becomes the subject for modulating the galactose content, e.g., a Chinese hamster ovary cell line (CHO) or a mouse myeloma cell line (NSO), but are not limited thereto.

Additionally, for modulating the galactosylation of monoclonal antibody, optimization of culture parameters, concentration of basal culture media, and further feeding of optimized culture media may be included.

That is, a method of modulating the galactosylation of monoclonal antibodies specified above according to the degree of modification requirement of the galactosylation may be applied together after determining the range of galactosylation for a desired monoclonal antibody and analyzing the galactosylation of the monoclonal antibody expressed by a cell line possessed.

Specifically, examples of the modification that can be embodied in operating an bioreactor for the production of monoclonal antibodies with modulated galactosylation may include optimization of culture parameters such as dissolved oxygen (DO), degree of acidity (pH), culture temperature, agitation, etc. Specifically, in the existing method, where the culture is performed at 36.5°C with 30% dissolved oxygen and pH 7.0 during the cell growth phase and at 30°C with 30% dissolved oxygen and pH 7.0 during the antibody-production phase, when the culture was performed after changing the temperature to 28°C, dissolved oxygen to 20%, and degree of acidity to pH 6.9, respectively, it was confirmed that modulating the galactosylation of monoclonal antibodies is possible.

Additionally, for the modulation of galactosylation, a basal culture medium may be concentrated to be used. Specifically, a culture medium which does not contain animal-derived components may be used, and a basal medium developed for a fed-batch culture may be optimized for modulating the galactosylation of monoclonal antibodies. Specifically, when media which were prepared by concentrating the basal culture medium, e.g., by 0.8-fold or 1.4-fold, were used in the main culture, it was possible to modulate the galactosylation of monoclonal antibodies.

Additionally, an feeding medium may be optimized for modulating the galactosylation. In the present invention, the galactosylation was reduced by modulating the concentration of an feeding medium and optimizing the concentration of metal components being added to the feeding medium.

The galactosylation of a target recombinant protein prepared by the above method may be measured by N-glycan profile analysis using the Q-TOF or UPLC device. The N-glycan profile analysis provides various information including G0F content, galactosylation index (GI), non-glycosylated heavy chain (NGHC), afucosylation %, high mannose contents, etc., and in the present invention, the change in G0F content was mostly mentioned.

Additionally, the method of the present invention may be one for preparing a population of antibodies with reduced galactosylation. That is, it is a method that can be applied when preparing a population of antibodies, which has a lower level of galactosylation than the analyzed value, after analyzing the galactosylation of monoclonal antibodies expressed by the cell line possessed.

As used herein, the term "population of antibodies" refers to a group of antibodies which includes antibodies that may have various glycan contents, and for the purpose of the present invention, the population of antibodies refers to an antibody group with modulated galactosylation, which includes galactosylated antibodies at a target ratio.

Another aspect of the present invention provides a method for modulating the galactosylation of a target recombinant protein, including increasing the osmolality of a culture solution of animal cells which express the target recombinant protein during the process of culturing the animal cells.

The method and each of the terms are the same as explained above.

Specifically, the method may include increasing the osmolality of a culture solution of animal cells which express a target recombinant protein on a particular day in culture during the process of culturing the animal cells, and the specific details are the same as explained above.

Still another aspect of the present invention provides a method for preparing a target recombinant protein with modulated galactosylation, including supplementing asparagine to a culture solution of animal cells which express the target recombinant protein during the process of culturing the animal cells.

The terms described are the same as explained above.

In the present invention, it was confirmed that when asparagine is supplemented to a culture solution of animal cells which express a target recombinant protein during the process of culturing the animal cells, the galactosylation of the target recombinant protein can be modulated.

Specifically, asparagine may be added in the form of an asparagine concentrate or a culture medium containing asparagine.

Additionally, the supplementation of asparagine may be performed multiple times during the process of culturing the animal cells which express the target recombinant protein at a particular time point of the culturing process. In particular, the supplementation of asparagine may be performed from day 4 to day 10, based on day 0 as the start of the main culture, and specifically, the supplementation of asparagine may be performed on all of day 4, day 7, and day 10, based on day 0 as the start of the main culture, thereby gradually increasing the concentration of asparagine in a culture solution.

When asparagine is supplemented a single time, there is a disadvantage in that the concentration of $NH_4^+$ in a culture solution rapidly increases and cell growth thereby becomes delayed, and eventually the expression level of the final protein is reduced. However, when asparagine is added in divided doses during the feeding according to the present invention, there is an advantage in that a desired amount of the protein can be produced while modulating the galactosylation without the above disadvantage.

The supplementation of asparagine may be performed so that the final concentration of asparagine in the culture solution of the animal cells can be in the range of 27.6 mM to 33.6 mM.

Specifically, asparagine may be supplemented so that the final concentration of asparagine in the culture solution of the animal cells can be 33.6 mM; for example, asparagine may be supplemented so that the final concentration of asparagine in the culture solution of the animal cells can be further increased by 6 mM to 18 mM, and more specifically, asparagine may be supplemented three times by sequentially adding asparagine at concentrations of 6 mM, 12 mM, and 18 mM, but is not limited thereto.

When the method is applied to animal cells, it induces the generation of ammonium ions ($NH_4^+$) thereby increasing the concentration of ammonium ions in cells. Accordingly, the pH of trans-golgi network increases and the activity of β-galactosyltransferase decreases, and the galactosylation of monoclonal antibody may thereby be inhibited.

Additionally, as described above, for modulating the galactosylation of monoclonal antibody, optimization of culture parameters, concentration of basal culture media, and further feeding of optimized culture media may be included.

That is, a method of modulating the galactosylation of monoclonal antibodies specified above according to the degree of modification requirement of the galactosylation may be applied together after determining the range of galactosylation for a desired monoclonal antibody and analyzing the galactosylation of the monoclonal antibody expressed by a cell line.

A further aspect of the present invention provides a method for modulating the galactosylation of a target recombinant protein, including supplementing asparagine to a culture solution of animal cells which express a target recombinant protein during the process of culturing the animal cells.

The method and terms described are the same as explained above.

A further aspect of the present invention provides a method for preparing a target recombinant protein with modulated galactosylation, including increasing the osmolality of a culture solution of animal cells which express the target recombinant protein, and supplementing asparagine to the culture solution during the process of culturing the animal cells.

The terms described are the same as explained above.

In the present invention, it was confirmed that when asparagine was added to a culture solution of animal cells which express the target recombinant protein while increasing the osmolality of the culture solution of the animal cells during the process of culturing the animal cells, the content of acidic antibody isomers, which can be decreased along with the increase in osmolality, was increased along with a simultaneous decrease in galactosylation according to the addition of asparagine.

In the above method, the increase of osmolality and the supplementation of asparagine may be performed simultaneously or sequentially. For example, the osmolality in a culture solution may be increased by a method such as supplementing sodium chloride followed by supplementing asparagine; or asparagine may be supplemented first and then the osmolality in a culture solution may be increased by adding sodium chloride or the like; or the supplementation of asparagine and the increase of osmolality in a culture solution may be applied simultaneously.

In particular, the increase of osmolality may be performed by applying the methods explained above.

The increase of osmolality may be performed to have the final osmolality in the culture solution to be in the range of 460 mOsm/kg to 500 mOsm/kg, and the supplementation of asparagine may be performed to supplement asparagine to adjust the final concentration of asparagine in the culture solution of animal cells to be in the range of 27.6 mM to 33.6 mM.

Additionally, the recombinant protein may be an antibody, specifically a monoclonal antibody, and the content of acidic antibody isomers of the population of antibodies prepared by the above method may be adjusted while simultaneously modulating the galactosylation of a target recombinant antibody by the method of the present invention. That is, galactosylation may be reduced according to the increase in osmolality, and simultaneously, the content of acidic antibody isomers, which can be reduced according to the increase in osmolality, may be increased by the addition of asparagine.

Accordingly, the above method has an advantage in that it can modulate the galactosylation of the population of the target antibody while simultaneously being capable of adjusting the content of acidic antibody isomers of the population of the target antibody.

The acidic antibody isomers are a kind of antibody isomer. An antibody isomer refers to an antibody in which a portion of the amino acids of the antibody with main activity is modified due to deamination or oxidation, and includes acidic antibody isomers and basic antibody isomers. Examples include an antibody isomer in which asparagine among amino acids is converted to aspartate by deamination, an antibody isomer in which methionine is converted into methionine sulfate by oxidation, etc. Additionally, when glutamate is present at the N-terminal of a heavy chain, the antibody isomer in which the glutamate is converted into pyroglutamate by forming a pentagon ring is included.

The analysis of these antibody isomers may be performed by chromatography, etc., and in the present invention, the analysis was performed by cation exchange resin chromatography. The content of acidic, main, and basic antibody isomers varies greatly according to the self-characteristics of monoclonal antibodies. The content of charge antibody isomers (charge variants) of monoclonal antibodies may vary according to the culture conditions of cell lines expressing the monoclonal antibodies (culture parameters, production media, etc.). Accordingly, the above method has an advantage in that it can adjust the desired galactose content while simultaneously adjusting the content of the acidic antibody isomers to the desired range.

### [DETAILED DESCRIPTION OF THE INVENTION]

Hereinafter, the present invention will be described in more detail with reference to the following Examples. However, these Examples are for illustrative purposes only, and the invention is not intended to be limited by these Examples.

### Example 1: Modulating galactosylation due to artificial increase of osmolality

There are various methods to increase osmolality of a culture solution during the progress of a fed-batch culture, e.g., a method of adding an excess amount of glucose to a culture solution, adding an aqueous sodium chloride solution to a culture solution, etc. In the present invention, as a representative method for increasing osmolality, a method of adding a 4 M aqueous sodium chloride solution to a culture solution was applied.

Explanation of preparation methods and media compositions

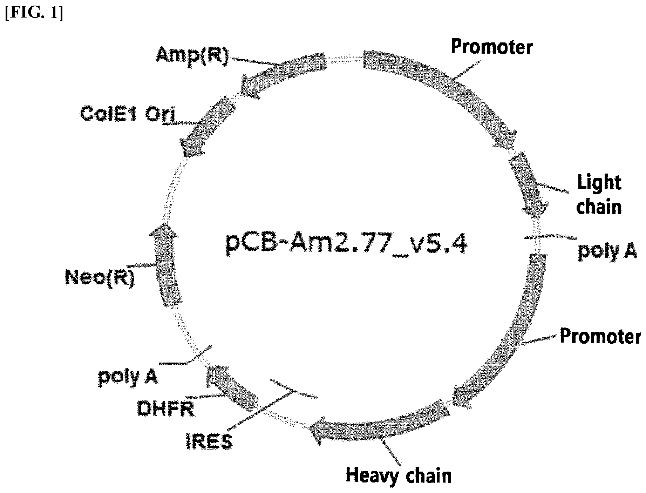

In the present invention, the monoclonal antibody to which the increase of osmolality of a culture solution or addition of_asparagine was applied is an adalimumab biosimilar antibody, and adalimumab biosimilar is a therapeutic agent for treating rheumatoid arthritis and Crohn's disease developed by Abbott. The adalimumab biosimilar DNA was produced by amplification via PCR referring to the amino acid sequences of the heavy chain and the light chain of the adalimumab antibody disclosed in U.S. Patent No. 6,090,382, and pCB-Am2.77_v5.4 (FIG. 1) was prepared using the promoter of pGL3 CUCBin, a vector developed by LG Life Sciences Ltd., which was then transfected into the CHO-DXB11 cell line, thereby preparing a cell line capable of expressing adalimumab biosimilar. The pCUCBin developed by LG Life Sciences Ltd. is one of the vectors disclosed in Korean Patent No. 10-1038126 (Novel hybrid promoter and recombinant vector which includes the promoter).

The media used in the present invention are three different kinds of a basal culture medium, a production medium (a main culture medium), and a feeding medium (an additional culture medium).

The basal culture medium is a medium used for the purpose of seed cultures. The production medium is a medium used in the culture performed for main antibody production (main culture) after seed culture, and may be prepared by concentrating a particular component(s) in a basal culture medium or adding a new component(s). In the present invention, a 1.4-fold concentrated basal culture medium was used as the production medium. The feeding medium is a culture medium being added for the purpose of promoting cell growth and increasing the amount of antibody expression during the culture. In the present invention, a 3.3-fold concentrated basal culture medium was used as the feeding medium. The basal culture medium, the production medium, and the feeding medium are all modified media of Iscove's Modified Dulbecco's Medium (IMDM) and the compositions of the culture media are shown in Table 1 below.

[Table 1] Compositions of basal culture medium

| Category | Components |
| --- | --- |
| IMDM modified medium | Merck 100131 |
| Buffer | HEPES |
| Buffer | Sodium bicarbonate |

(continued)

| Category | Components |
| --- | --- |
| Carbon source | Glucose |
| Nitrogen source | Glutamine |
| etc. | Sodium chloride |
| Growth hormone | Insulin |
| etc. | MTX |
| Vitamin | L-ascorbic acid |
| Vitamin | Biotin |
| Vitamin | Choline chloride |
| Vitamin | Folic acid |
| Peptone | Sheff CHO plus pf acf (Kerry) |
| Metal | Boric acid |
| Metal | Cobalt chloride hexahydrate |
| Metal | Copper sulfate pentahydrate |
| Metal | Ferric citrate |
| Metal | Magnesium chloride anhydrous |
| Metal | Manganese sulfate monohydrate |
| Metal | Potassium nitrate |
| Metal | Sodium selenite pentahydrate |
| Metal | Zinc sulfate heptahydrate |

The method of antibody production used in the present invention is a process for culturing animal cells using a bioreactor, and the culturing of animal cells is a process for growing animal cells in a culture medium and enabling the animal cells to express antibodies by a particular treatment (e.g., lowering temperature). The culture methods variously include a batch culture, a fed-batch culture, a continuous culture, a perfusion culture, etc., and the culture method used in the present invention for culturing the adalimumab biosimilar cell line is a fed-batch culture. The fed-batch culture is a culture method which progresses in such a manner that an additional culture medium is added at least once or twice on a particular day in culture while performing a production culture.

The fed-batch culture method used in Examples is to commonly further add an additional culture medium in an amount corresponding to 5% of the volume of the current culture solution of a culture medium four times on day 1, day 4, day 7, and day 10 in culture.

Example 1.1: Change in galactosylation according to the time point of increase in osmolality

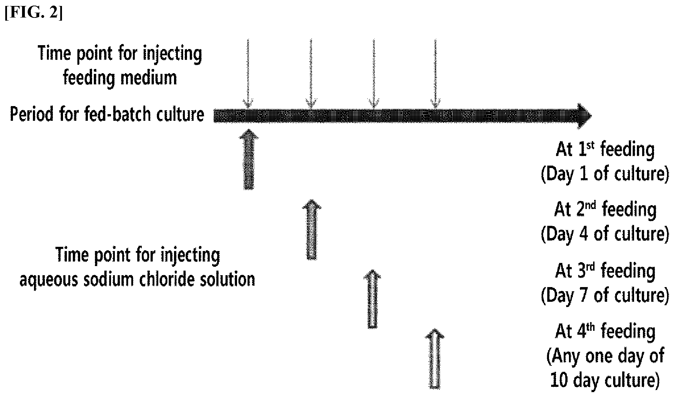

A fed-batch culture was performed on a real 30 mL scale using a 250 mL shake flask. The feeding strategy of the fed-batch culture and the time point of adding an aqueous sodium chloride solution were the same as illustrated in FIG. 2. The culture broth started with an osmolality of 320 mOsm/kg and was artificially increased to 430 mOms/kg by feeding with a 4 M aqueous sodium chloride (NaCl) solution, thereby obtaining the final osmolality of 450 mOms/kg. Regarding the additional culture medium, the first feeding was performed on day 1 in culture, and a further feeding was performed at every 3 days thereafter for a total of four feedings. Among the four feedings, the 4 M aqueous sodium chloride solution was added once or four times in divided doses (osmolality is gradually increased during the culture period) to increase the osmolality of the culture broth. Upon completion of the culture, the expression amount and galactosylation of monoclonal antibody were analyzed and the results are shown in Table 2 below.

[Table 2] Change in antibody quality according to the time point of increase in osmolality

| Culture Conditions | Antibody Titer (mg/L) | G0F (%)* | Acidic (%) | Final Osmolality (mOsm/kg) |
|---|---|---|---|---|
| Gradual increase of osmolality during culture period | 1392.0 | 60.4 | 20.1 | 453.0 |
| At 1st feeding (day 1 of culture) osmolality increased | 1215.5 | 66.1 | 17.6 | 445.0 |
| At 2nd feeding (day 4 of culture) osmolality increased | 1268.3 | 68.8 | 16.7 | 451.0 |
| At 3rd feeding (day 7 of culture) osmolality increased | 1438.7 | 67.1 | 16.4 | 451.0 |
| At 4th feeding (day 10 of culture) osmolality increased | 1580.1 | 66.2 | 17.4 | 454.0 |
| Osmolality not increased | 1771.8 | 63.5 | 18.5 | 364.0 |

$$* \quad \overline{G0F(\%) = G0F/(G0F+G1F+G2F)}$$

As a result, as shown in Table 2, there was a difference in the galactosylation of monoclonal antibody according to the time point of feeding the 4 M aqueous sodium chloride solution. In the case of a shake flask culture, it is not easy to directly adjust the dissolved oxygen (DO) and degree of acidity (pH) during the culture process, and thus there may not be a big difference in G0F among different experimental conditions. However, in the culture being performed in a bioreactor, the dissolved oxygen and degree of acidity can be directly adjusted, and thus the difference in G0F may be maximized.

Example 1.2: Change in galactosylation of monoclonal antibody according to the change in the range of increase in osmolality

Example 1.1 showed that the galactosylation of monoclonal antibody can vary according to the time point of increasing the osmolality of a culture broth. When the feeding of the aqueous sodium chloride solution was performed at the 2nd feeding (day 4 of culture) and the 3rd feeding (day 7 of culture) of an additional feeding medium, the G0F increased maximally. Additionally, a secondary fed-batch culture using a shaker flask was performed in order to examine the effect of the difference in the increasing level of osmolality in the culture broth on the galactosylation of monoclonal antibody. The osmolality at the initial stage of culture was 320 mOsm/kg, and the culture was performed by adding a varied amount of a 4 M aqueous sodium chloride solution to a culture solution on day 4 or day 7 of culture according to the targeted increase of osmolality in a manner similar to the primary shake flask culture, and the expression amount and galactosylation of the monoclonal antibody were analyzed.

[Table 3] Change in antibody quality according to the difference in the range of increase in osmolality

| Culture Conditions | Targeted Osmolality (mOsm/kg) | Antibody Titer (mg/L) | G0F (%)* | Acidic (%) | Final Osmolality (mOsm/kg) |
|---|---|---|---|---|---|
| Osmolality not increased | X | 1136.2 | 67.4 | 15.6 | 365.0 |
| At 2nd feeding (day 4 of culture) osmolality increased | Up to 400 | 948.9 | 70.4 | 15.0 | 424.0 |
| | Up to 440 | 824.1 | 71.0 | 14.6 | 460.0 |
| | Up to 480 | 573.7 | 67.9 | 14.5 | 505.0 |
| | Up to 520 | 543.8 | 66.5 | 13.9 | 539.0 |

(continued)

| Culture Conditions | Targeted Osmolality (mOsm/kg) | Antibody Titer (mg/L) | G0F (%)* | Acidic (%) | Final Osmolality (mOsm/kg) |
|---|---|---|---|---|---|
| At 3rd feeding (day 7 of culture) osmolality increased | Up to 400 | 1053.4 | 70.5 | 14.9 | 416.0 |
| | Up to 440 | 927.4 | 69.5 | 14.5 | 454.0 |
| | Up to 480 | 851.1 | 68.5 | 14.3 | 497.0 |
| | Up to 520 | 775.1 | 66.9 | 14.2 | 533.0 |

*
$$G0F(\%) = G0F/(G0F+G1F+G2F)$$

According to the increased level of osmolality of the culture solution, the galactosylation of the monoclonal antibody appeared to vary according to the level of increase in osmolality. When the osmolality of the culture broth was increased to 480 mOsm/kg or higher during the culture progress, the content of G0F rather decreased. That is, there was an appropriate range of osmolality for maximally increasing the G0F content of the monoclonal antibody (increased up to 400 mOsm/kg to 480 mOsm/kg during the culture progress).

Example 1.3: Confirmation experiments in a bioreactor

As a result of performing confirmation experiments in a bioreactor based on the results of the primary and secondary shake flask cultures, the change in the galactosylation of monoclonal antibody according to the increasing conditions of osmolality was confirmed.

The culture was performed after intentionally increasing the osmolality to 440 mOsm/kg, 500 mOsm/kg, and 523 mOsm/kg by adding a 4 M aqueous sodium chloride (NaCl) solution to a culture broth in a 1.4 L bioreactor with 1 L working volume and at the 2nd feeding (day 4 of culture), respectively.

[Table 4] Change in antibody quality according to the difference in the range of increase in osmolality in a bioreactor

| Condition for Increasing Osmolality (mOsm/kg) at the 2nd Feeding | Antibody Titer (mg/L) | G0F (%)* | Acidic (%) | NH₄⁺ Conc. (mM) | Final Osmolality (mOsm/kg) |
|---|---|---|---|---|---|
| Not increased | 1980.6 | 63.7 | 26.5 | 3.6 | 321 |
| Increased up to 440 | 1740.2 | 71.8 | 18.4 | 7.7 | 479 |
| Increased up to 500 | 1361.6 | 58.3 | 18.4 | 7.3 | 549 |
| Increased up to 523 | 1361.7 | 54.8 | 19.3 | 5.6 | 567 |

*
$$G0F(\%) = G0F/(G0F+G1F+G2F)$$

When the osmolality was increased to 500 mOsm/kg or higher at the 2nd feeding (final osmolality of 549 mOsm/kg and 567 mOsm/kg), the G0F content rather decreased. In an experiment in a bioreactor where the osmolality was increased to 440 mOsm/kg at the 2nd feeding, the G0F content was 71.8%.

**Example 2: Production of excess ammonium ions in the culture solution by the addition of asparagine**

According to the exprerimental results (Table 4), when an excess amount of asparagine was added to a culture solution, the concentration of ammonium ions ($NH_4^+$) within the culture solution increased significantly. The galactosylation of monoclonal antibody can be indirectly modulated by a method of increasing the concentration of ammonium ions within the culture solution by adding an excess amount of asparagine.

[Table 5] Increase of ammonium according to the addition of asparagine in culture broth

| Culture Conditions | Conc. of Added Asparagine (mM) | Final Conc. of Ammonium Ions (mM) |
|---|---|---|
| Asparagine (not added) | 0 | 3.4 |
| Asparagine (added) | 18.9 | 8.1 |

Example 2.1: A fed-batch culture with addition of an asparagine concentrate

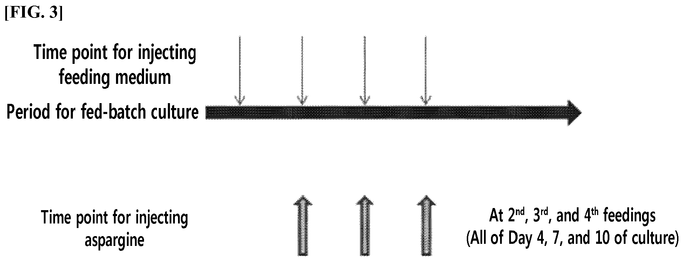

A fed-batch culture was performed in a real 30 mL scale using a 250 mL shake flask. The feeding strategy of the fed-batch culture and the time point of adding an aqueous sodium chloride solution were the same as illustrated in FIG. 3. The asparagine concentrate was prepared at a concentration of 200 mM. The time point of injecting an additional culture medium was day 1, day 4, day 7, and day 10 of culture, and the addition of the asparagine concentrate was performed at every feeding from the time point of the 2nd feeding (day 4 of culture) (a total of 3 times). Upon completion of the culture, the expression amount and galactosylation of monoclonal antibody were analyzed.

[Table 6] Change in galactosylation of monoclonal antibody according to the addition of asparagine

| Culture Conditions | | | Antibody Titer (mg/L) | G0F (%)* | Acidic (%) | Final Conc. of $NH_4^+$ (mM) |
|---|---|---|---|---|---|---|
| Production medium | Conc. of asparagine added per one feeding (mM) | Conc. of total asparagine added (mM) | | | | |
| 1 X | 0 | 0 | 1738.5 | 56.3 | 25.9 | 4.1 |
| 1 X | 1 | 3 | 1705.8 | 59.9 | 21.7 | 5.2 |
| 1 X | 2 | 6 | 1535.2 | 61.4 | 19.2 | 6.7 |
| 1 X | 4 | 12 | 1500.0 | 64.3 | 19.3 | 8.8 |
| 1.4 X | 0 | 0 | 1729.9 | 60.2 | 21.1 | 5.6 |
| 1.4 X | 1 | 3 | 1721.0 | 61.0 | 21.3 | 7.4 |
| 1.4 X | 2 | 6 | 1588.5 | 61.5 | 20.1 | 7.3 |
| 1.4 X | 4 | 12 | 1563.7 | 61.9 | 19.3 | 9.0 |

*
$$G0F(\%) = G0F/(G0F+G1F+G2F)$$

The G0F content increased as asparagine was added to the culture solution by feeding. As the concentration of asparagine being added increased, the final concentration of $NH_4^+$ in the culture solution increased as well. The increase range of the G0F content by the injection of asparagine in a 1X production medium was bigger than that in a 1.4X production medium. When asparagine is added all at once at the beginning of the culture, the $NH_4^+$ concentration becomes high from the beginning of the culture and thus delays cell growth, and eventually lowers the amount of expression of final antibodies. Accordingly, a method of adding a divided dose of asparagine at the time of feeding was employed.

Example 2.2: Feeding of an additional culture medium containing an excess amount of asparagine

In a fed-batch culture using a bioreactor, unlike the shake flask fed-batch culture, an asparagine concentrate was not prepared separately but included in the additional culture medium. Based on the plan to supplement an additional 6 mM asparagine at feedings, an additional culture medium containing 120 mM asparagine was prepared and feeding was performed using the same. The culture was performed in a 1.4 L bioreactor with 1 L working volume, and the expression amount and galactosylation of monoclonal antibody were analyzed.

[Table 7] Bioreactor experiment using an additional culture medium with addition of an excess amount of asparagine

| Culture Conditions | Antibody Titer (mg/L) | G0F (%) | Acidic (%) | $NH_4^+$ (mM) | Final Osmolality (mOsm/kg) |
|---|---|---|---|---|---|
| Asparagine (not added) | 1980.6 | 63.7 | 26.5 | 3.6 | 321. |
| Addition of 6 mM of asparagine at feedings from the 2nd feeding (a total of 18 mM added) | 1281.8 | 73.9 | 20.6 | 13.5 | 379 |

The batch in which an additional culture medium with addition of an excess amount of asparagine was used showed a 10.2% increase in G0F content. Considering that the $NH_4^+$ concentration in the culture solution with addition of an excess amount of asparagine increased by 9.9 mM, the decrease of the galactosylation of monoclonal antibody was confirmed to be due to the change in the $NH_4^+$ concentration in the culture solution. The osmolality increased in the culture solution with addition of an excess amount of_asparagine by 58 mOsm/kg.

Example 3: Combination of an increase of osmolality and addition of asparagine

The G0F of monoclonal antibody could be increased by an intentional increase of osmolality or addition of asparagine during a fed-batch culture. The effect of the combined application of the increase of osmolality and the addition of_asparagine on the galactosylation of monoclonal antibody and charge antibody isomers (charge variants) was examined.

Example 3.1: Experiment of a simultaneous application of increasing osmolality and adding asparagine in a bioreactor

A fed-batch culture was performed in a 1.4 L bioreactor with 1 L working volume by simultaneously applying the increase of osmolality and the addition of asparagine. In the case of a bioreactor, in which the increase of osmolality and the addition of_asparagine were applied simultaneously, the osmolality of a culture broth was increased up to 423 mOsm/kg, considering the effect of increasing osmolality by the addition of asparagine (addition of 120 mM asparagine to an additional culture medium). Upon completion of the culture, the expression amount and galactosylation of monoclonal antibody were analyzed.

[Table 8] Change in galactosylation by a simultaneous application of increasing osmolality and adding asparagine in a bioreactor

| Culture Conditions | Antibody Titer (mg/L) | G0F (%) | Acidic (%) | $NH_4^+$ (mM) | Final Osmolality (mOsm/kg) |
|---|---|---|---|---|---|
| Increased up to osmolality of 450 | 1511.6 | 73.6 | 20.6 | 8.80 | 502.0 |
| Increased up to osmolality of 423 + addition of asparagine | 1548.1 | 75.4 | 23.1 | 10.5 | 490.0 |

The difference in G0F between the two different conditions, i.e., a condition in which only osmolality was applied and a condition in which a combination of osmolality and asparagine was applied, was not large, but the G0F content was further increased. When asparagine was added to a culture broth, the percentage of acidic antibody isomers (acidic variants) was decreased compared to the cultured broth without addition of asparagine (Tables 6 and 7). However, when a combination of osmolality and asparagine was added to a culture broth, the acidic antibody isomers (acidic variants) rather increased.

For increasing the G0F content, methods such as an increase of osmolality, addition of asparagine, and addition of asparagine simultaneously with an increase of osmolality may be applied. In particular, for increasing the percentage of the acidic antibody isomers (acidic variants) among the charge profiles as well as the G0F content, it appears to be more useful to use a strategy applying both the increase of osmolality and the addition of asparagine at the same time.

Those of ordinary skill in the art will recognize that the present invention may be embodied in other specific forms without departing from its spirit or essential characteristics. The described embodiments are to be considered in all respects only as illustrative and not restrictive. The scope of the present invention is therefore indicated by the appended claims rather than by the foregoing description. All changes which come within the meaning and range of equivalency of the claims are to be embraced within the scope of the present invention.

**Claims**

1. A method for preparing a target recombinant protein with modulated galactosylation, comprising increasing the osmolality of a culture solution of animal cells, which express a target recombinant protein, on a particular day in culture during the process of culturing the animal cells

2. The method of claim 1, wherein the culturing is performed by a fed-batch culture.

3. The method of claim 1, wherein the increase of the osmolality is performed on any one day between day 1 and day 10 of a main culture, based on day 0 as the start of the main culture.

4. The method of claim 1, wherein the increase of the osmolality is performed on any one day among day 1, day 4, day 7, and day 10, based on day 0 as the start of the main culture.

5. The method of claim 4, wherein the increase of the osmolality is performed on day 4 or day 7, based on day 0 as the start of the main culture.

6. The method of claim 1, wherein the increase of the osmolality is performed to adjust the final osmolality in the culture solution to be in the range of 460 mOsm/kg to 500 mOsm/kg.

7. The method of claim 1, wherein the increase of the osmolality is performed by at least one method selected from the group consisting of a method of supplementing sodium chloride or potassium chloride to the culture solution of the animal cells, and a method of adding glucose to the culture solution.

8. The method of claim 1, wherein the recombinant protein is an antibody.

9. The method of claim 8, wherein the method is for preparing a population of antibodies with reduced galactosylation.

10. The method of claim 1, wherein the increase of the osmolality is performed a single time during the process of culturing the animal cells, which express the target recombinant protein.

11. A method for modulating the galactosylation of a target recombinant protein, comprising increasing the osmolality of a culture solution of animal cells, which express the target recombinant protein, on a particular day during the process of culturing the animal cells.

12. A method for preparing a target recombinant protein with modulated galactosylation, comprising supplementing asparagine to a culture solutionof animal cells, which express the target recombinant protein, during the process of culturing the animal cells.

13. The method of claim 12, wherein the asparagine is in the form of an asparagine concentrate or a culture medium comprising asparagine.

14. The method of claim 12, wherein the supplementing asparagine is performed multiple times during the process of culturing the animal cells, which express the target recombinant protein, at a particular time point of the culturing process.

15. The method of claim 14, wherein the supplementation of asparagine is performed from day 4 to day 10, based on day 0 as the start of the main culture.

16. The method of claim 14, wherein the supplementation of asparagine is performed on day 4, day 7, and day 10, based on day 0 as the start of the main culture.

17. The method of claim 12, wherein the supplementation of asparagine is performed by supplementing asparagine in order to adjust the final concentration of asparagine in the culture solution of the animal cells to be in the range of 27.6 mM to 33.6 mM.

18. The method of claim 17, wherein the supplementation of asparagine is performed by supplementing asparagine in order to adjust the final concentration of the asparagine in the culture solution of the animal cells to be 33.6 mM.

19. The method of claim 12 or 17, wherein the supplementation of asparagine is performed by supplementing asparagine three times in order to adjust the final concentration of the asparagine in the culture solution of the animal cells to be in the sequential order of 6 mM, 12 mM, and 18 mM.

20. A method for modulating the galactosylation of a target recombinant protein, comprising supplementing asparagine to a culture solution of animal cells, which express the target recombinant protein, during the process of culturing the animal cells.

21. A method for preparing a target recombinant protein with modulated galactosylation, comprising increasing the osmolality of a culture solution of animal cells, which express the target recombinant protein, and supplementing asparagine to the culture solution, during the process of culturing the animal cells.

22. The method of claim 21, wherein the increase of the osmolality and the supplementation of asparagine are performed simultaneously or sequentially.

23. The method of claim 21, wherein the increase of the osmolality is performed by at least one method selected from the group consisting of a method of supplementing sodium chloride or potassium chloride to the culture solution of animal cells and a method of adding glucose to the culture solution.

24. The method of claim 21, wherein the increasing increase of the osmolality is performed to have the final osmolality in the culture solution to be in the range of 460 mOsm/kg to 500 mOsm/kg.

25. The method of claim 21, wherein the supplementation of asparagine is to supplement asparagine to adjust the final concentration of asparagine in the culture solution of animal cells to be in the range of 27.6 mM to 33.6 mM.

26. The method of claim 21, wherein the recombinant protein is an antibody.

27. The method of claim 26, wherein the method is to adjust the galactosylation of the target recombinant antibody and to adjust the content of acidic antibody isomers (acidic variants) of the population of antibodies prepared by the method.

[FIG. 1]

[FIG. 2]

Time point for injecting
feeding medium

Period for fed-batch culture

At 1st feeding
(Day 1 of culture)

At 2nd feeding
(Day 4 of culture)

Time point for injecting
aqueous sodium chloride solution

At 3rd feeding
(Day 7 of culture)

At 4th feeding
(Any one day of
10 day culture)

[FIG. 3]

Time point for injecting
feeding medium

Period for fed-batch culture

Time point for injecting
aspargine

At 2nd, 3rd, and 4th feedings
(All of Day 4, 7, and 10 of culture)

## INTERNATIONAL SEARCH REPORT

| | International application No. |
|---|---|
| | **PCT/KR2015/000997** |

**A. CLASSIFICATION OF SUBJECT MATTER**

*C12N 5/02(2006.01)i, C12N 5/071(2010.01)i, C07K 16/18(2006.01)i*

According to International Patent Classification (IPC) or to both national classification and IPC

**B. FIELDS SEARCHED**

Minimum documentation searched (classification system followed by classification symbols)
C12N 5/02; C12N 5/10; C12N 5/00; C12N 15/00; C12N 5/071; C07K 16/18

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: recombinant protein, cultivation, osmotic pressure, galactosylation, asparagine

**C. DOCUMENTS CONSIDERED TO BE RELEVANT**

| Category* | Citation of document, with indication, where appropriate, of the relevant passages | Relevant to claim No. |
|---|---|---|
| A | GRAMER, MICHAEL J. et al., "Modulation of antibody galactosylation through feeding of uridine, manganese chloride, and galactose", Biotechnology and Bioengineering, 2011, vol. 108, no. 7, pages 1591-1602<br>See abstract; pages 1594-1599; figures 1-8; and table 1. | 1-27 |
| A | US 2012-0276631 A1 (BENGEA, Cornelia T. et al.) 01 November 2012<br>See abstract and claims 1-15. | 1-27 |
| A | WO 2011-019619 A1 (GENENTECH, INC.) 17 February 2011<br>See abstract and claims 1-40. | 1-27 |
| A | KR 10-2008-0048556 A (CELLCA GMBH) 02 June 2008<br>See abstract and claims 1-46. | 1-27 |
| A | WO 01-59089 A2 (GENENTECH, INC.) 16 August 2001<br>See abstract and claims 1-9. | 1-27 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 APRIL 2015 (29.04.2015) | **29 APRIL 2015 (29.04.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

INTERNATIONAL SEARCH REPORT
Information on patent family members

International application No.

**PCT/KR2015/000997**

| Patent document cited in search report | Publication date | Patent family member | Publication date |
|---|---|---|---|
| US 2012-0276631 A1 | 01/11/2012 | EP 2702077 A2<br>US 2015-0050693 A1<br>WO 2012-149197 A2<br>WO 2012-149197 A3 | 05/03/2014<br>19/02/2015<br>01/11/2012<br>10/05/2013 |
| WO 2011-019619 A1 | 17/02/2011 | AU 2010-282733 A1<br>CN 102498206 A<br>CN 104059955 A<br>EP 2464725 A1<br>JP 2013-501522 A<br>KR 10-2012-0088667 A<br>US 2011-0091936 A1<br>US 2014-0024078 A1<br>US 8512983 B2 | 01/03/2012<br>13/06/2012<br>24/09/2014<br>20/06/2012<br>17/01/2013<br>08/08/2012<br>21/04/2011<br>23/01/2014<br>20/08/2013 |
| KR 10-2008-0048556 A | 02/06/2008 | EP 1931765 A2<br>JP 2009-509514 A<br>US 2008-0254513 A1<br>US 8426202 B2<br>WO 2007-036291 A2<br>WO 2007-036291 A3 | 18/06/2008<br>12/03/2009<br>16/10/2008<br>23/04/2013<br>05/04/2007<br>26/07/2007 |
| WO 01-59089 A2 | 16/08/2001 | EP 1254216 A2<br>EP 1254216 B1<br>JP 2003-521942 A<br>US 2003-0211573 A1<br>US 2007-0111284 A1<br>WO 01-59089 A3 | 06/11/2002<br>07/06/2006<br>22/07/2003<br>13/11/2003<br>17/05/2007<br>04/07/2002 |

Form PCT/ISA/210 (patent family annex) (July 2009)

**EP 3 101 120 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120276631 A
- US 6090382 A
- KR 101038126